# EUROPEAN PATENT SPECIFICATION

(11) **EP 4 052 687 B1**
(45) Date of publication and mention of the grant of the patent: **14.05.2025**
(21) Application number: 21161054.8
(22) Date of filing: 05.03.2021
(51) Int. Cl.: A61F 13/20

(54) **IMPROVED APPARATUS FOR SHAPING THE INTRODUCTORY END OF A TAMPON AND METHOD USING SUCH APPARATUS**
VERBESSERTE VORRICHTUNG ZUM FORMEN DES EINFÜHRUNGSENDES EINES TAMPONS UND VERFAHREN ZUR VERWENDUNG EINER SOLCHEN VORRICHTUNG
APPAREIL AMÉLIORÉ POUR FORMER L'EXTRÉMITÉ D'INTRODUCTION D'UN TAMPON ET PROCÉDÉ UTILISANT UN TEL APPAREIL

(43) Date of publication of application: 07.09.2022
(73) Proprietor: Ontex BV, 9255 Buggenhout (BE); Ontex Group NV, 9320 Erembodegem (BE)
(72) Inventor: Heege, Thomas, 56761 Düngenheim (DE); Krause, Daniel, 56075 Koblenz (DE)
(74) Representative: Saurat, Thibault

(56) References cited:
- EP-A1- 0 797 971
- US-A- 4 816 100

## Description

### TECHNICAL FIELD

The invention pertains to the technical field of manufacturing tampon blanks, tampons, specifically digital tampons, and intermediate products thereof. The invention relates to an improved apparatus for shaping the introductory end of a tampon, and a method using such apparatus, as well as the product manufactured by said method and apparatus.

### BACKGROUND

Tampons are well known in the art and are used for feminine hygiene. As such, many tampon manufacturing methods and apparatuses have been disclosed in the prior art. Generally, a distinction is made between folded and rolled tampons. The former has improved absorbent characteristics but possess less strength and are commonly used with an applicator to reduce the chance of tears and other damages to the tampon before insertion. Rolled tampons are slightly less absorbent, but sturdier and can be applied digitally, as opposed to the folded tampons. Furthermore, measures can be taken to increase the absorbency of the rolled tampons.

Tampons comprise a rolled or folded absorbent fiber sheet and a withdrawal string for removal. Tampons are manufactured by rolling or folding multiple-layers. The multiple-layered sheets comprise a layer of absorbent material of a certain length, upon which a strip of web material is bonded which has only a fraction of the length of the layer of the absorbent material, thus creating the multiple-layered sheets.

It is desired to attain a maximal production speed, but one recurring problem is that the machines are often limited in speed during the head-shaping step, as most machines exploit a heating press to impart pressure and heat onto the introductory end of the tampon. These machines are not efficient enough as it requires several hits to correctly shape a tampon head and these machines are often subject to mechanical failure with parasite melting of the thermoplastic film onto the heating plates. For example, US 2014/0115847 describes a process with a compressor to form the head. US 4,816,100 also describes a process where a heated forming die is pressed onto the tampon blank in order to shape the introductory end. The issue in these processes is that the head-shaping step takes too much time because the quality of the heat-shaping depends on the temperature of the heating element and the duration of the heat-sealing step. More specifically, the quality is improved if heat-shaping is performed at lower temperature and for a longer duration, thus lowering production speed.

The invention aims to provide an apparatus and method which ensures a fast, efficient, reliable and cleaner shaping of the introductory end of a tampon.

### SUMMARY OF THE INVENTION

The present invention provides an apparatus for shaping the introductory end of a tampon, comprising:
- a carrying pipe for holding the tampon;
- a support wheel that carries a plurality of carrying pipes, the support wheel comprising conveying means to transport each carrying pipe; and
- a head-shaping station for shaping the introductory end of the tampon.

According to the invention, the head-shaping station comprising an ultrasonic device, the ultrasonic device comprising at least one head-shaping sonotrode emitting ultrasonic vibrations at a frequency between 10 kHz and 70 kHz, preferably between 20 kHz and 50 kHz, preferably a frequency of 35 kHz, the ultrasonic device comprises a first and a second head-shaping sonotrodes emitting ultrasonic vibrations at a frequency between 10 kHz and 70 kHz, preferably between 20 kHz and 50 kHz, preferably a frequency of 35 kHz.

The ultrasonic device emitting ultrasonic vibrations enables to increase the number of hits to the tampon introductory end, when comparing to a conventional heating press, during a same amount of time. In other words, for the same amount of time, a ultrasonic device enables to get more hits to the tampon head in comparison to a convention heating press, thus the introductory end will be better shaped. Also, a conventional heating press requires 5 or 6 moulds to hit the tampon introductory end enough times to obtain a proper shaping of the head, whereas with an ultrasonic device, it is possible to reduce this number of moulds. This enable to improve the production yield, since the tampons can move quicker to a following stage, e.g. the wrapping step, where the tampon is covered by a wrapper.

According to an embodiment, the first and second head-shaping sonotrodes are aligned on an arcuate axis.

According to an embodiment, the head-shaping station comprises an actuation mechanism enabling the movement of the ultrasonic device closer or remoter to the support wheel and the tampon.

According to an embodiment, the apparatus comprises a pushing mechanism comprising a plate with at least one protruding peg to press against the trailing end of the tampon that is opposite to the introductory end.

According to an embodiment, the first and second head-shaping sonotrodes have different cross sectional profiles.

According to an embodiment, the head-shaping sonotrode has a cavity that is ogive shaped.

The invention also pertains to a method for shaping the introductory end of a tampon using the apparatus as described above, comprising the steps of:
- placing a tampon inside a carrying pipe;
- conveying said carrying pipe and tampon to a head-shaping station;
- shaping the introductory end of the tampon;
- conveying said tampon away from the shaping station.

According to the invention, the shaping of the introductory end of the tampon is done with an ultrasonic device, the ultrasonic device comprising at least one head-shaping sonotrode emitting ultrasonic vibrations at a frequency between 10 kHz and 70 kHz, preferably between 20 kHz and 50 kHz, preferably a frequency of 35 kHz, during a lapse of time which is comprised between 0,1 and 1 s, preferably between 0,2 and 0,5 s, the ultrasonic device comprises a first and a second head-shaping sonotrode emitting ultrasonic vibrations at a frequency between 10 kHz and 70 kHz, preferably between 20 kHz and 50 kHz, preferably a frequency of 35 kHz, during a lapse of time which is comprised between 0,1 and 1 s, preferably between 0,2 and 0,5 s, the tampon being shaped by the first head-shaping sonotrode then subsequently by the second head-shaping sonotrode.

According to an embodiment, the shaping of the introductory end of the tampon further comprises the sub-steps:
- the head-shaping sonotrode and the peg slide forth, preferably simultaneously, towards the tampon, the head-shaping sonotrode slides to abut against the tampon's head and the first peg slides to abut against the trailing end of the tampon;
- the head-shaping sonotrode emits ultrasonic vibrations at a frequency between 10 kHz and 70 kHz, preferably between 20 kHz and 50 kHz, preferably a frequency of 35 kHz, during a lapse of time which is comprised between 0,1 and 1 s, preferably between 0,2 and 0,5 s;
- the head-shaping sonotrode and the peg slide back, preferably simultaneously, away for the head-shaped tampon.

According to an embodiment, the shaping of the introductory end of the tampon further comprises the sub-steps:
- the peg slides forth within the carrying pipe and abuts against the compressed tampon and pushes said tampon up against the head-shaping sonotrode which is in a stationary position;
- the head-shaping sonotrode applies ultrasonic vibrations to the tampon introductory end at a frequency between 10 kHz and 70 kHz, preferably between 20 kHz and 50 kHz, preferably a frequency of 35 kHz, during a lapse of time which is comprised between 0,1 and 1 s, preferably between 0,2 and 0,5 s;

- the peg slides back and the head-shaping sonotrode slides forth so that the head-shaping sonotrode can push the tampon back to its initial position in the carrying pipe;
- the head-shaping sonotrode slides backwards to its stationary position.

According to an embodiment, the method further comprises the sub-steps:
- the introductory end of the tampon is shaped by the first head-shaping sonotrode using the sub-steps described above;
- the carrying pipe and tampon are conveyed to the second head-shaping sonotrode position;
- the introductory end of the tampon is shaped by the second head-shaping sonotrode using the sub-steps described above, the sub-steps used for the first and second head-shaping sonotrode being different.

All of these embodiments mentioned above can be taken individually or in combination.

Further embodiments are described below and in the claims.

### DESCRIPTION OF FIGURES

**FIG. 1** illustrates, according to a view in perspective, a module, or a part, of an apparatus to compress and shape the introductory end of a tampon in accordance with an embodiment of the present invention.
**FIG. 2** illustrates a close-up of the module in Figure 1, according to a different view in perspective, namely the head-shaping station.
**FIG. 3** illustrates a pushing mechanism of a head-shaping station in a partly disassembled view.
**FIG. 4** illustrates a close-up of a head-shaping ultrasonic device according to a view in perspective.
**FIG. 5** illustrates a cross section profile of a sonotrode according to an embodiment of the invention.
**FIG. 6** illustrates a tampon after having its introductory end being shaped by an apparatus according to an embodiment of the invention.
**FIG. 7** schematically illustrates a first head-shaping sub-step, from a top view or a side view.
**FIG. 8** schematically illustrates a second head-shaping sub-step subsequent to the first sub-step illustrated in FIG. 7, from a top view or a side view.
**FIG. 9** schematically illustrates a third head-shaping sub-step subsequent to the second sub-step illustrated in FIG. 8, from a top view or a side view.
**FIG. 10** schematically illustrates a fourth head-shaping sub-step subsequent to the third sub-step illustrated in FIG. 9, from a top view or a side view.

### DETAILED DESCRIPTION OF THE INVENTION

The present invention concerns an improved apparatus for shaping the introductory end of a tampon, and a method using such apparatus, as well as the product manufactured by said method and apparatus.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The term "emit ultrasonic vibrations" is equivalent to the term "apply ultrasonic vibrations to".

The terms "form" and "shape" are equivalent and means the spatial form or contour of an element and "shaping" means imparting a particular form or shape to an element, in particular the introductory end of a tampon.

The term "introductory end" equivalent to the term "head" means the end of the tampon that is designed to be inserted first in a body cavity. It corresponds to the end that is opposite of the end that presents the withdrawal string.

The term "trailing end" equivalent to the term "rear" means the end of the tampon that is designed to be inserted last in a body cavity. It corresponds to the end of that tampon that presents the withdrawal string.

The term "carrying pipe" means a housing with a tubular design that has a hollow inner space, or cavity, in order to receive tampon within said hollow inner space. In other words the carrying pipe corresponds to a hollow elongated cylinder.

The terms "sonotrode" and "head-shaping sonotrode" are equivalent.

The term "convex crown area" or "convex dome apex" designates a surface shape where each single point on the surface of the summit can be connected with each other point on the surface of the summit by means of lines running within the summit.

The term "nonwoven web material" means a sheet material having a structure of individual fibers or threads which are interlaid, but not in a regular manner such as occurs with knitting or weaving processes. Nonwoven fabrics or webs have been formed from many processes such as for example, melt blowing processes, spun bonding processes, and bonded carded web processes.

The term "thermoplastic" is meant to describe a material that softens when exposed to heat and which substantially returns to its original condition when cooled to room temperature.

The term "rayon" refers to a manufactured regenerated cellulose fiber, made from purified cellulose. It has a smooth, soft surface, and is therefore very suitable to be used in a tampon.

The term "concurrently" or "essentially concurrently" refers to the simultaneous or overlapping occurrence or execution of two or more events or steps.

The recitation of numerical ranges by endpoints includes all numbers and fractions subsumed within that range, as well as the recited endpoints.

A process for making a tampon comprises several steps. For example, the process comprises a bonding step where a first web material, for example an absorbent material such as rayon, viscose or cotton is bonded with a second web material, for example a thermoplastic film or a nonwoven web material. The process also comprises a winding and sealing step where the first and second web materials are winded and sealed together to form a tampon pre-blank. The tampon pre-blank is then compressed in a press. The compressed tampon is then transferred to the head-shaping apparatus according to the invention where the introductory end of the tampon is shaped. The tampon is then conveyed to a wrapping station where it is covered by a wrapper. Of course, the process can comprise additional steps such as the deposit of a withdrawal string and its knotting; the steps can be in this order or in another. The focus of the present invention pertains to a method for shaping the head, or introductory end, of a tampon and the apparatus to implement such method.

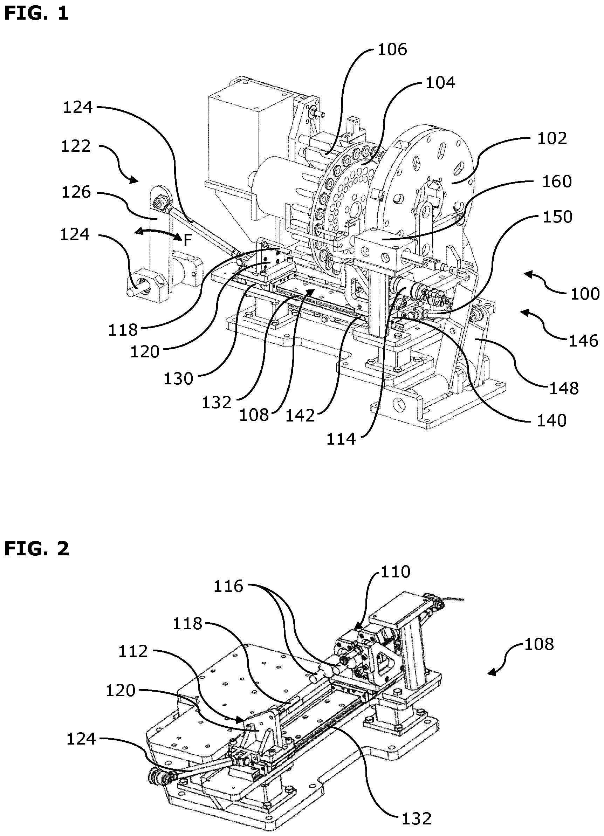

FIG.1 illustrates a head shaping apparatus 100 according to the invention. The tampon pre-blank is transferred from a winding and sealing station (not illustrated) to a press 102. The press 102 comprises a plurality of arms, for example 4, 6, 8 or 12 that press the tampon pre-blank into a compressed tampon with grooves on its outer surface. Each compressed tampon is then transferred to a support wheel 104 that comprises a plurality of carrying pipes 106, or hollow tubes, each compressed tampon being placed in a carrying pipe 106. Each carrying pipe 106 is hollow and extends in a longitudinal axis and has two open ends, one at each longitudinal end. Each compressed tampon is introduced within a carrying pipe 106, namely within the hollow space defined by the cylindric walls of the carrying pipe 106, through one open-end of the carrying pipe 106. The support wheel 104 comprises actuation means to rotate: for example a motor or engine can rotate a shaft to make the support wheel 104 turn. The support wheel 104, and therefore every carrying pipes 106 carried by the support wheel 104, rotate in a clockwise or counter-clockwise direction, here in a clockwise direction, to guide the carrying pipe 106 towards the head-shaping station 108.

FIG. 2 illustrates a close-up of the head-shaping station 108, the support wheel 104 not being illustrated in this view for clarity reasons. The head-shaping station 108 comprises an ultrasonic device 110 and a pushing mechanism 112. The ultrasonic device 110 comprises at least one vibration generator 114 (illustrated on FIG. 1), for example and not limited to, a magnetostrictive or piezoelectric transducer. Said generator is attached to at least one tapering rod or probe usually made of metal, such as and not limited to titanium, aluminum or steel, that will be referenced herein as the head-shaping sonotrode 116. Each head-shaping sonotrode 116 is submitted to the vibrations generated by a vibration generator 114 and said head-shaping sonotrode 116 then emits or applies this vibrational energy to the head of the compressed tampon 162. The pushing mechanism 112 comprises a peg 118 that is narrower than the hollow carrying pipe 106, said peg 118 being attached to a plate 120. In other words, the peg 118, corresponding here to a cylindrical finger that has a diameter that is smaller than the diameter of the carrying pipe 106, in this manner is able to slide within the interior space of the carrying pipe 106 and press against the tampon 162.

The plate 120 is attached to an actuating mechanism 122 (illustrated FIG.1) that can move the plate 120 back-and-forth. In other words, the actuating mechanism 122 is preferably a reciprocating mechanism imparting a back-and-forth linear motion. Such actuation mechanisms can comprise for example and not limited to, scotch yoke, slider-crank, piston or pneumatic engine, traction elevator geared or gearless, turbine, spring.

As illustrated here, the actuating mechanism 122 corresponds here to a crank mechanism combined with a rod. More specifically, the actuating mechanism 122 comprises an arm 124 that is fixed to the plate 120 through a reversible or permanent fixation. As illustrated here, the actuating mechanism 122 comprises a lever 126 which rotates in a back-and-forth motion F through the impulse of a shaft 128 that is rotated itself by a motor (not illustrated). The lever 126 is linked to the arm 124 so that said arm 124 also moves in a back-and-forth motion. The arm 124 being linked to the plate 120 also forces it to move in a back-and-forth reciprocate manner. The peg 118 being attached to the plate 120 also moves in the same back-and-forth movements as the plate 120. The invention is not limited to this embodiment and other means of actuation can be considered as long as they induce a back-and-forth motion.

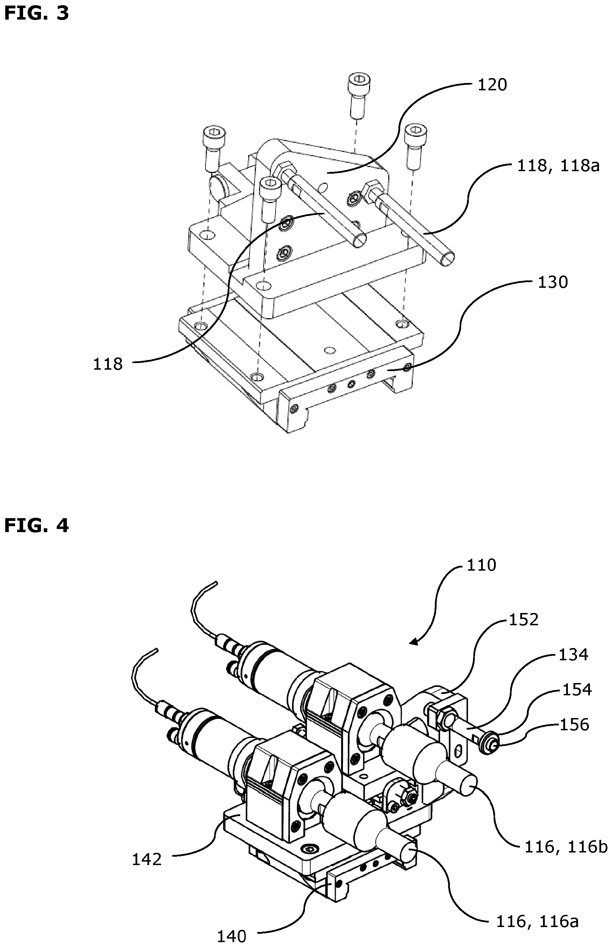

Preferably, the peg 118 and the plate 120 moves in a linear back and forth motion. In an embodiment, the plate 120 is carried by a sliding base 130 (as illustrated in FIG. 3) that slides within a railing 132, or rail tracks, (illustrated in FIG. 1 OR FIG. 2) so as to restrain the movement of the plate 120 and peg to a linear reciprocating motion. The invention is not limited to this embodiment, for example the plate 120 can be attached to a sliding base comprising at least one peg that slides within a cam track.

The amplitude of the movement of the plate and peg is limited by the rotation of the lever 126, the length of the arm 124. In addition or alternatively, the railing 132 can comprise stop ends to define a limit to the railing or cam track and restrict the movement of the plate 120 and pegs 118.

As illustrated in FIG. 3, a plurality of pegs 118, here two, are carried by the plate 120. Of course, the invention is not limited to this embodiment and the number of pegs carried by a plate 120 can vary from 1 to 2, 3, 4, 5, 6, 7, 8, 9, 10, 11, 12 or more pegs 118. Each peg 118 can form a continuity of matter with the plate 120 or be reversibly attached to the plate 120 as illustrated here, this way it is possible to adapt the number of pegs in accordance with the number of functional head-shaping sonotrodes 116. Each peg 118 corresponds to a right or straight cylinder that extends in a longitudinal direction, namely, each peg 118 extends in the same direction as the railing 132 or the reciprocating direction imparted by the actuating mechanism 122. Each peg 118 has one end fixed or forming continuity of matter with the plate 120 and the opposed end that is either flat, chamfered, convex or concave. The pegs 118 can be identical to one another or have different shapes and/or dimensions.

As explained above, the diameter of the peg is smaller than the diameter of the hollow carrying pipe 106 so that when the pegs 118 are in motion thanks to the actuating mechanism 122, they slide within the hollow space of the carrying pipe 106.

As explained above, the number of pegs 118 on the plate 120 corresponds to the number of functional head-shaping sonotrodes 116 present at the head-shaping station 108. In the example illustrated in FIG. 2, the head-shaping station 108, specifically the ultrasonic device 110, comprises two head-shaping sonotrodes 116. Of course, the invention is not limited to said number of sonotrodes and the head-shaping station 108 can comprise 2, 3, 4, 5, 6, 7, 8, 9, 10 or more sonotrodes. Of course, the head-shaping process lasts for a certain amount of time. Not shaping enough the head 172, or introductory end, of a tampon 162 can lead to a product being unfinished and/or uncomfortable to insert, while having too many head-shaping steps causes a loss of time and reduces the production yield. It is a balance between a proper introductory end shape and manufacturing yield. By using a head-shaping station 108 comprising an ultrasonic device 110, the inventors have discovered a surprising effect whereby the sonotrode is able to apply more hits to the compressed tampon pre-blanks thus enabling a better head shape or design when applying less time or the same amount of time as for a conventional heated hammer. This improvement enables to reduce the number of moulds where five or six moulds are conventionally used for heat-plate hammers. With this improved apparatus 100, it is possible to obtain a better result with two ultrasonic moulds or sonotrodes 116. Secondly, the usage of ultrasonic bonding has also the significant benefit that it does not provoke any undesired melting of the film which can occur when using heat-plate hammers because of radiant heating phenomena, resulting in a cleaner process.

As recited hereinabove, each compressed tampon pre-blank is transferred from the press 102 to a hollow carrying pipe 106, specifically within the interior space of the carrying pipe 106. The support wheel 104 then rotates in a clockwise direction so that the compressed tampon pre-blank reaches the head-shaping station 108. At that point, the peg 118 slides within the hollow carrying pipe and pushes the compressed tampon 162 against the head-shaping sonotrode 116. Each head-shaping sonotrode 116 applies ultrasonic vibrations at a frequency between 10 kHz and 70 kHz, preferably between 20 kHz and 50 kHz, preferably 35 kHz, to the introductory end 172 of the tampon 162. The introductory end 172 of the tampon 162 is thereby shaped in a dome-like configuration in order to have a shape that is more comfortable to insert in a body cavity.

FIG. 4 illustrates a close-up of an embodiment of the ultrasonic device 110 according to the invention. The ultrasonic device 110 comprises here two head-shaping sonotrodes 116 and a pusher 134. The two sonotrodes 116 and the pusher 134 are aligned in this order along an arcuate axis, following the same direction and radius as the support wheel 104, and the angle between the sonotrodes 116 or the sonotrode in the middle 116 and the pusher 134 corresponds to the same angle between two carrying pipes 106. The angle between two carrying pipes 106 is comprised between 5° and 45°, for example between 10° and 30°, for example is about 15°. The first sonotrode 116a corresponds to the pre-shaping sonotrode whereas the second sonotrode 116b corresponds to the final shaping sonotrode.

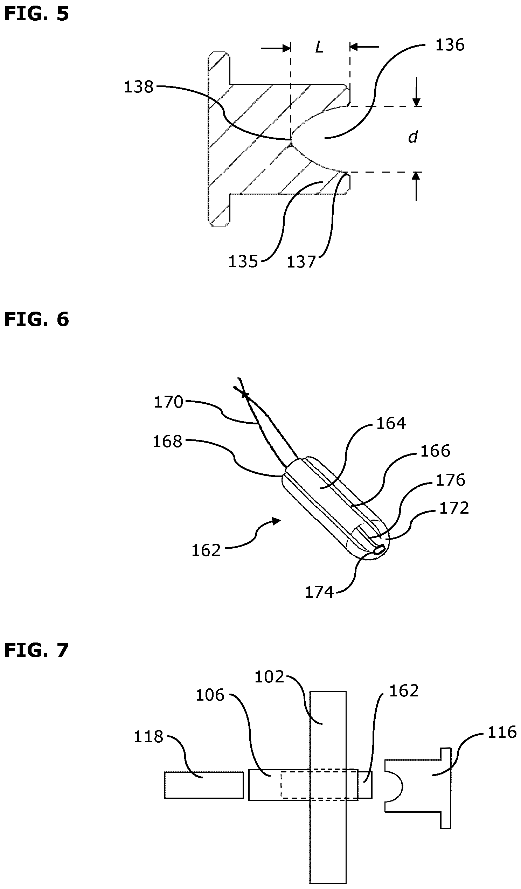

FIG. 5 illustrates a cross-section of a sonotrode 116 in a side or top view. The head-shaping sonotrode 116 corresponds to a metal rod with an excavation at one end thus defining two branches 135 and a cavity 136 in between the two branches in a cross-sectional profile. Each sonotrode 116 has a cavity 136 that is ogive shaped with a diameter d and a length L. The diameter d of the cavity 136 is comprised between 6 and 20 mm, preferably between 10 and 15 mm, more preferably the diameter d is 12 mm. The diameter d of the cavity 136 corresponds substantially to the diameter of the tampon 162. The length L of the cavity 136 is comprised between 6 and 18 mm, preferably between 8 and 12 mm, more preferably the length L is 10 mm. Here the length L of the cavity 136 is smaller than the diameter d of the cavity 136. Such dimensions are particularly suited to define a well-shaped introductory end 172 for the tampon 162. The extremities of the branches 135 can present a chamfer 137, or bevel, on the inner side, i.e. the side adjacent to the cavity 136, in order to have more clearance when the introductory end 172 of the tampon is introduced within the cavity 136 of the sonotrode 116. The cavity 136 can have other profile, for example it can have a half, or truncated, squircle profile.

The tip, or extremity, 138 of the ogive-shaped cavity 136 can be round shaped so that the tampon can have a dome-shaped meaning a convex dome apex or convex crown area. The tip 138 of the cavity 136 corresponds to the central part of the cavity 136, or the point where the two branches 135 start to branch out as illustrated in FIG. 5. For example, the cavity 136 can be in the form of a hemispherical dome so as to shape a tampon with an introductory end 172 with a hemispherical dome profile. The tip 138 of the ogive-shape cavity 136 can have a flat portion at its end so the tampon displays a truncated domed-shape introductory end. The tip 138 of the ogive-shape cavity 136 can also be convex so as to form a tampon 162 with a concave dome apex at its introductory end 172 as illustrated in FIG. 6. To sum up, the cavity 136 can have different nose cone profile for example hemispherical, elliptical, tangent, spherical blunted tangent, *etc.* depending on the desired final shape.

The first and second sonotrode 116a, 116b can have the same profile, or shape, and/or dimensions, to ensure that the introductory end 172 of the tampon 162 is identical.

Alternatively, the two sonotrodes 116 can have different profiles, or shapes, and/or dimensions so that the second sonotrode 116b can make a fine tuning of the introductory end. For example, the cavity of the second sonotrode 116b can have the same diameter d as the first sonotrode 116a but a smaller length L than the cavity of the first sonotrode 116a in order to shape only a part of the introductory end 172.

In the embodiment where the first and second sonotrode 116a,116b have different dimensions, the pegs 118 preferably have different dimensions so that a peg 118 is adapted to the specific length of the corresponding sonotrode 116. For example, if the second sonotrode 116b has a smaller length L than the first sonotrode 116a, than the first peg 118a will have a longer length than the second peg 118b to compensate this length gap. Alternatively, the first and second pegs 118a,118b can have the same length and the second sonotrode 116b can be slightly shifted towards the support wheel 104 and the carrying pipe 106 to compensate the length difference, or in other words, the second sonotrode 116b is slightly shifted in a direction that is orthogonal to the axis on which the sonotrodes are aligned.

According to an embodiment not illustrated, ribs can also be placed within the cavity in order to form additional grooves 176 on the tampon head.

Preferably, the ultrasonic device 110 also moves in a linear back-and-forth motion. In an embodiment, the ultrasonic device 110 is carried by a sliding base 140 (as illustrated in FIG. 4) that slides within a railing, or rail tracks, so to restrain the movement of the ultrasonic device 110 to a linear reciprocating motion. Preferably, the sliding base 140 slides within the same railing as the sliding base 130 of the actuating mechanism 122. As illustrated in FIG. 1 and FIG. 2, the railing 132 enables the sliding of both the sliding base 130 carrying the plate 120 and pegs 118 and the sliding base 140 carrying the ultrasonic device 110. The invention is not limited to this embodiment, for example the ultrasonic device 110 can be attached to a sliding base comprising at least one peg that slides within a cam track. The ultrasonic device 110 can be carried directly by the sliding base 140, or it can be carried by a plate 142 that is fixed to the sliding base 140 as illustrated in FIG. 4.

The plate 142 is attached to an actuation mechanism 146 (illustrated in FIG.1) that can move the plate 142 back-and-forth. The actuation mechanism 146 is as described above, preferably a reciprocating mechanism inducing a back-and-forth linear motion. The actuation mechanism 146 for the ultrasonic device 110, as the actuating mechanism 122 of the plate 120 and pegs 118, comprises a lever 148 which rotates in a back-and-forth motion through the impulse of a shaft that is rotated itself by a motor (not illustrated). The lever 148 is linked to an arm 150 so that said arm 150 also moves in a back-and-forth motion. The arm 150 being linked to the plate 142 also forces it to move in a back-and-forth reciprocate manner. The ultrasonic device 110 being attached to the plate 142 also moves in the same back-and-forth movements as the plate 142. The invention is not limited to this embodiment and other means of actuation can be considered as long as they induce a back-and-forth motion.

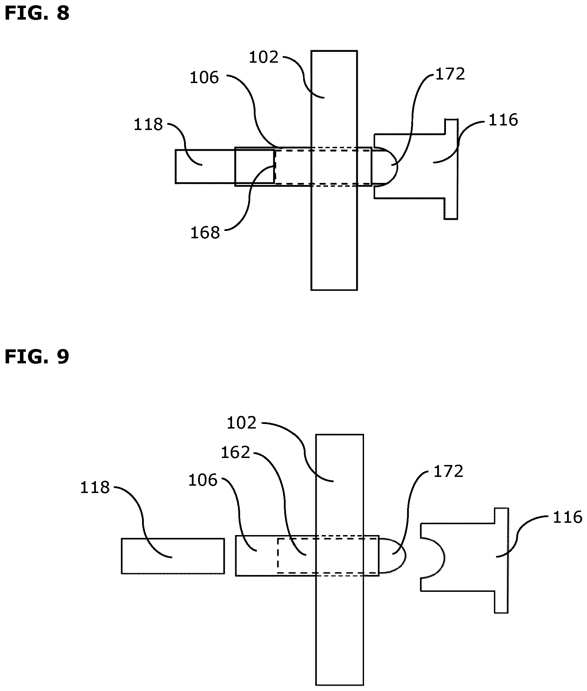

A method for shaping the introductory end of a tampon 162 according to the invention comprises the following steps as illustrated in FIG. 7 through 9. Each tampon 162, after being compressed in the press 102, is placed inside a carrying pipe 106, preferably with a portion of the tampon 162 sticking out of the carrying pipe 106. The support wheel 104 rotates and the carrying pipe 106 and tampon 162 are then conveyed to the head-shaping station 108, more specifically up to the first sonotrode 116a location as schematically illustrated in FIG. 7. The introductory end 172 of the tampon 162 is then shaped at this location. More specifically, as schematically illustrated in FIG. 8 the first sonotrode 116a and the first peg 118a slide forth, preferably simultaneously, towards the support wheel 104, the carrying pipe 106 and the tampon 162. The first sonotrode 116a slides to abut against the tampon's head 172 and the first peg 118a slides to abut against the trailing end 168 of the tampon 162 and counters the force applied by the first sonotrode 116a. The first sonotrodes 116a then emits ultrasonic vibrations at a frequency between 10 kHz and 70 kHz, preferably between 20 kHz and 50 kHz, preferably 35 kHz, during a lapse of time which is comprised between 0,1 and 1 s, preferably between 0,2 and 0,5 s, for example 0,3 s. The first sonotrode 116a and then the first peg 118a slide back, preferably concurrently, away for the support wheel 104, the carrying pipe 106 and the head-shaped tampon 162 as illustrated in FIG. 9. The support wheel 104 then rotates again and coveys the carrying pipe 106 and the shaped tampon 162 towards the second sonotrode 116b. According to this embodiment, the pushing mechanisms 112, i.e. peg 118, and the ultrasonic device 110, i.e. sonotrode 116, preferably simultaneously slide back-and-forth in opposite linear direction; this enables to improve the yield time.

The invention is not limited to the previous embodiment. For example, according to another embodiment which is not illustrated, each compressed tampon arranged in a carrying pipe 106, is transported up to the first sonotrode 116a location. The first peg 118a is moved by the actuating mechanism 122 and slides forth within the carrying pipe 106. The first peg 118a abuts against the compressed tampon 162, specifically the trailing end 168 of the tampon 162, and pushes said tampon 162 up against the first sonotrode 116a specifically within the cavity 136 of the sonotrode 116 which stays stationary at this point. The first sonotrode 116a applies ultrasonic vibrations to the tampon introductory end 172 at a frequency between 10 kHz and 70 kHz, preferably between 20 kHz and 50 kHz, preferably 35 kHz, during a lapse of time which is comprised between 0,1 and 1 s, preferably between 0,2 and 0,5 s, for example 0,3 s. The peg 118 and the sonotrode 116 then slide concurrently towards the plate 120 so that the sonotrode 116 can push the tampon 162 back to its previous position in the carrying pipe 106. In other words, the sonotrode 116 slides forth towards the support wheel 104 whereas the peg 118 slides back away from the support wheel 104. The sonotrode 116 then slides backwards to its stationary position. This embodiment enables that the tampon 162 does not need to have a protruding portion sticking out from the carrying pipe 106 before reaching the head-shaping station 108 thus limiting contamination risks.

After the tampon introductory end has been shaped by the first sonotrode 116a, the support wheel 104 rotates and the carrying pipe 106 and tampon 162 are transferred to the second sonotrode 116b location. The tampon 162 can undergo the same process steps as described above for both sonotrodes. Alternatively, the tampon 162 can undergo the process steps described in the first illustrated embodiment for the first sonotrode and the alternative process steps for the second sonotrode or vice versa.

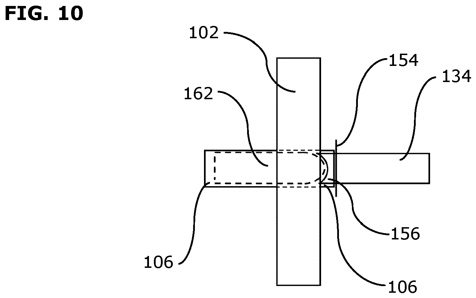

After the tampon introductory end has been shaped by the second sonotrode 116b, the support wheel 104 rotates and the carrying pipe 106 and head-shaped tampon 162 are transferred to the pusher 134 location. As illustrated in FIG. 4, the pusher 134 corresponds to a cylinder or peg with a washer 154 at one end and the other end is fixed to, or forms continuity of matter with, a connecting rod 152 that is linked to the plate 142 and incidentally the sliding base 140 so as to move simultaneously in the same back and forth motion as the sonotrodes 116. The end with a washer 154 has a portion 156 of the cylinder or peg that protrudes from the washer 154. The diameter of the washer 154 is superior to the diameter of the carrying pipe 106 and the diameter of the pusher 134 and in particular of said portion 156 is substantially equal to the diameter of the tampon, thus inferior to the diameter of the carrying pipe 106. The pusher 134 slides forth and pushes back the head-shaped tampon 162 within the carrying pipe 106 up to the point where the washer 154 abuts against the carrying pipe 106 as illustrated in FIG. 10. The end with the portion 156 can also have a concave profile to push the tampon 162 without deforming the introductory end 172 as illustrated in FIG. 10. The pusher slides back afterwards according to the movement induced by the sliding base 140 and the actuation mechanism 146. This step enables the tampon 162 to be rightfully positioned within the carrying pipe 106 with no portion protruding out of the carrying pipe 106 thus limiting contamination risks during the transfer of the tampon 162 to the wrapping station.

The support wheel 104 rotates and the carrying pipe 106 is transferred to a transfer device 160 location. The transfer device 160 corresponds to a device that can push or pull the tampon 162 out of the carrying pipe 106 onwards to the wrapping station where the tampon 162 is covered by a wrapper. In other words, the process comprises a step where the tampon 162 is ejected from the carrying pipe 106.

FIG. 6 illustrates a tampon 162 after having been shaped by the first and second sonotrodes 116a, 116b. The tampon 162 comprises a cylindrical body 164 extending in a longitudinal direction. Incidentally the carrying pipe 106 is a hollow cylindrical body extending in the same longitudinal direction and that has a larger diameter than the tampon 162 so that said tampon 162 can be placed within the inner space of the carrying pipe 106. On its outer surface, the tampon 162 can comprise grooves 166 to improve fluid absorption. As illustrated in FIG. 6, the tampon 162 comprises a withdrawal string 170 on its rear or trailing end 168. The trailing end 168 corresponds to the longitudinal end of tampon that is opposite to the longitudinal end that is shaped by the sonotrodes 116. The head, or introductory end, 172 shaped by the sonotrodes 116 has a dome like configuration. It can present a concave apex area 174, if the tip 138 of the ogive-shape cavity 136 is convex. Alternatively, the head 172 of the tampon can have a convex dome apex or convex crown area for example in the form or shape of a hemispherical dome. The introductory end 172 can comprise one or more additional grooves 176 should one or more ribs be arranged within the sonotrode cavity 136.

It is supposed that the present invention is not restricted to any form of realization described previously and that some modifications can be added to the presented example of fabrication without reappraisal of the appended claims.

## Claims

1. Apparatus (100) for shaping the introductory end (172) of a tampon (162), comprising:
- a carrying pipe (106) for holding the tampon (162);
- a support wheel (104) that carries a plurality of carrying pipes (106), the support wheel (104) comprising conveying means to transport each carrying pipe (106); and
- a head-shaping station (108) for shaping the introductory end (172) of the tampon (162);
the head-shaping station (108) comprising an ultrasonic device (110), the ultrasonic device (110) comprising at least one head-shaping sonotrode (116) emitting ultrasonic vibrations at a frequency between 10 kHz and 70 kHz, preferably between 20 kHz and 50 kHz, preferably 35 kHz,
**characterized in that** the ultrasonic device (110) comprises a first and a second head-shaping sonotrode (116a, 116b) emitting ultrasonic vibrations at a frequency between 10 kHz and 70 kHz, preferably between 20 kHz and 50 kHz, preferably 35 kHz.

2. Apparatus (100) according to claim 1, **characterized in that** the first and second head-shaping sonotrodes (116a, 116b) are aligned on an arcuate axis.

3. Apparatus according to claim 1 or 2, **characterized in that** the head-shaping station (108) comprises an actuation mechanism (146) enabling the movement of the ultrasonic device (110) closer or remoter to the support wheel (104) and the tampon (162).

4. Apparatus according to the preceding claim 3, wherein the apparatus (100) comprises a pushing mechanism (112) comprising a plate (120) with at least one protruding peg (118) to press against the trailing-end (168) of the tampon (162) that is opposite to the introductory end (172).

5. Apparatus according to any of the preceding claims, wherein the first and second head-shaping sonotrodes (116a,116b) have different cross sectional profiles.

6. Apparatus according to any of the preceding claims, wherein the head-shaping sonotrode (116) has a cavity (136) that is ogive shaped.

7. Method for shaping the introductory end of a tampon using the apparatus (100) according to any of the preceding claims, comprising the steps of:
a. placing a tampon (162) inside a carrying pipe (106);
b. conveying said carrying pipe (106) and tampon (162) to a head-shaping station (108);
c. shaping the introductory end (172) of the tampon (162).
d. conveying said tampon (162) away from the head-shaping station (108).
**characterized in that** the shaping of the introductory end (172) of the tampon (162) is done with an ultrasonic device (110),
the ultrasonic device (110) comprising at least one head-shaping sonotrode (116) emitting ultrasonic vibrations at a frequency between 10 kHz and 70 kHz, preferably between 20 kHz and 50 kHz, preferably 35 kHz, during a lapse of time which is comprised between 0,1 and 1 s, preferably between 0,2 and 0,5 s,
**characterized in that** the ultrasonic device (110) comprises a first and a second head-shaping sonotrode (116a, 116b) emitting ultrasonic vibrations at a frequency between 10 kHz and 70 kHz, preferably between 20 kHz and 50 kHz, preferably 35 kHz, during a lapse of time which is comprised between 0,1 and 1 s, preferably between 0,2 and 0,5 s, the tampon (162) being shaped by the first head-shaping sonotrode (116a) then subsequently by the second head-shaping sonotrode (116b).

8. Method according to the preceding claim, **characterized in that** the shaping of the introductory end (172) of the tampon (168), step (c), further comprises the sub-steps:
a. the head-shaping sonotrode (116,116a,116b) and the peg (118,118a,118b) slide forth towards the tampon (162), the head-shaping sonotrode (116,116a,116b) slides to abut against the tampon's head (172) and the first peg (118,118a,118b) slides to abut against the trailing end (168) of the tampon;
b. the head-shaping sonotrode (116,116a,116b) emits ultrasonic vibrations at a frequency between 10 kHz and 70 kHz, preferably between 20 kHz and 50 kHz, preferably 35 kHz, during a lapse of time which is comprised between 0,1 and 1 s, preferably between 0,2 and 0,5 s.
c. the head-shaping sonotrode (116,116a,116b) and the peg (118,118a,118b) slide back away for the head-shaped tampon (162).

9. Method according to claim 7, **characterized in that** the shaping of the introductory end (172) of the tampon (168), step (c), further comprises the sub-steps:
a. the peg (118,118a,118b) slides forth within the carrying pipe (106) and abuts against the compressed tampon (162) and pushes said tampon (162) up against the head-shaping sonotrode (116,116a,116b) which is in a stationary position;
b. the head-shaping sonotrode (116,116a,116b) applies ultrasonic vibrations to the tampon introductory end (172) at a frequency between 10 kHz and 70 kHz, preferably between 20 kHz and 50 kHz, preferably 35 KHz, during a lapse of time which is comprised between 0,1 and 1 s, preferably between 0,2 and 0,5 s;
c. the peg (118,118a,118b) slides back and the head-shaping sonotrode (116,116a,116b) slides forth so that the head-shaping sonotrode (116,116a,116b) pushes the tampon (162) back to its initial position in the carrying pipe (106);
d. the head-shaping sonotrode (116,116a,116b) slides backwards to its stationary position.

10. Method according to claims 8 or 9, where:
a. the introductory end (172) of the tampon (162) is shaped by the first head-shaping sonotrode (116a) using the sub-steps described in claim 12 or 13;
b. the carrying pipe (106) and tampon (162) are conveyed to the second head-shaping sonotrode (116b) position;
c. the introductory end (172) of the tampon (162) is shaped by the second head-shaping sonotrode (116b) using the sub-steps described in claim 8 or 9, the sub-steps used for the first and second head-shaping sonotrode (116a,116b) being different.

## Patentansprüche

1. Vorrichtung (100) zum Formen des Einführendes (172) eines Tampons (162), umfassend:
- ein Trägerrohr (106) zum Halten des Tampons (162);
- ein Stützrad (104), das eine Vielzahl von Trägerrohren (106) trägt, wobei das Stützrad (104) Fördermittel zum Transportieren jedes Trägerrohrs (106) umfasst; und
- eine Kopfformungsstation (108) zum Formen des Einführendes (172) des Tampons (162);
wobei die Kopfformungsstation (108) eine Ultraschallvorrichtung (110) umfasst, wobei die Ultraschallvorrichtung (110) mindestens eine kopfformende Sonotrode (116) umfasst, die Ultraschallschwingungen mit einer Frequenz zwischen 10 kHz und 70 kHz, vorzugsweise zwischen 20 kHz und 50 kHz, vorzugsweise von 35 kHz, aussendet,
**dadurch gekennzeichnet, dass** die Ultraschallvorrichtung (110) eine erste und eine zweite kopfformende Sonotrode (116a, 116b) umfasst, die Ultraschallschwingungen mit einer Frequenz zwischen 10 kHz und 70 kHz, vorzugsweise zwischen 20 kHz und 50 kHz, vorzugsweise von 35 kHz, aussenden.

2. Vorrichtung (100) nach Anspruch 1, **dadurch gekennzeichnet, dass** die erste und die zweite kopfformende Sonotrode (116a, 116b) auf einer bogenförmigen Achse ausgerichtet sind.

3. Vorrichtung nach Anspruch 1 oder 2, **dadurch gekennzeichnet, dass** die Kopfformungsstation (108) einen Betätigungsmechanismus (146) umfasst, der es ermöglicht, die Ultraschallvorrichtung (110) näher an das Stützrad (104) und den Tampon (162) heran zu bewegen oder weiter davon zu entfernen.

4. Vorrichtung nach dem vorstehenden Anspruch 3, wobei die Vorrichtung (100) einen Schiebemechanismus (112) umfasst, der eine Platte (120) mit mindestens einem hervorstehenden Stift (118) umfasst, um gegen das hintere Ende (168) des Tampons (162) zu drücken, das dem Einführende (172) gegenüberliegt.

5. Vorrichtung nach einem der vorstehenden Ansprüche, wobei die erste und die zweite kopfformende Sonotrode (116a, 116b) unterschiedliche Querschnittsprofile aufweisen.

6. Vorrichtung nach einem der vorstehenden Ansprüche, wobei die kopfformende Sonotrode (116) einen Hohlraum (136) aufweist, der ogivenförmig ist.

7. Verfahren zum Formen des Einführendes eines Tampons unter Verwendung der Vorrichtung (100) nach einem der vorstehenden Ansprüche, umfassend die Schritte:
a. Platzieren eines Tampons (162) innerhalb eines Trägerrohrs (106);
b. Befördern des Trägerrohrs (106) und des Tampons (162) zu einer Kopfformungsstation (108);
c. Formen des Einführendes (172) des Tampons (162);
d. Abtransportieren des Tampons (162) von der Kopfformungsstation (108);
**dadurch gekennzeichnet, dass** das Formen des Einführendes (172) des Tampons (162) mit einer Ultraschallvorrichtung (110) erfolgt,
wobei die Ultraschallvorrichtung (110) mindestens eine kopfformende Sonotrode (116) umfasst, die Ultraschallschwingungen mit einer Frequenz zwischen 10 kHz und 70 kHz, vorzugsweise zwischen 20 kHz und 50 kHz, vorzugsweise von 35 kHz, während einer Zeitspanne aussendet, die zwischen 0,1 und 1 s, vorzugsweise zwischen 0,2 und 0,5 s, liegt,
**dadurch gekennzeichnet, dass** die Ultraschallvorrichtung (110) eine erste und eine zweite kopfformende Sonotrode (116a, 116b) umfasst, die Ultraschallschwingungen mit einer Frequenz zwischen 10 kHz und 70 kHz, vorzugsweise zwischen 20 kHz und 50 kHz, vorzugsweise von 35 kHz, während einer Zeitspanne aussenden, die zwischen 0,1 und 1 s, vorzugsweise zwischen 0,2 und 0,5 s, liegt, wobei der Tampon (162) durch die erste kopfformende Sonotrode (116a) und anschließend durch die zweite kopfformende Sonotrode (116b) geformt wird.

8. Verfahren nach dem vorstehenden Anspruch,
**dadurch gekennzeichnet, dass** das Formen des Einführendes (172) des Tampons (168), Schritt (c), ferner die Teilschritte umfasst:
a. die kopfformende Sonotrode (116, 116a, 116b) und der Stift (118, 118a, 118b) gleiten nach vorn in Richtung des Tampons (162), die kopfformende Sonotrode (116, 116a, 116b) gleitet, bis sie an den Kopf (172) des Tampons stößt, und der erste Stift (118, 118a, 118b) gleitet, bis er an das hintere Ende (168) des Tampons stößt;
b. die kopfformende Sonotrode (116, 116a, 116b) sendet Ultraschallschwingungen mit einer Frequenz zwischen 10 kHz und 70 kHz, vorzugsweise zwischen 20 kHz und 50 kHz, vorzugsweise von 35 kHz, während einer Zeitspanne aus, die zwischen 0,1 und 1 s, vorzugsweise zwischen 0,2 und 0,5 s, liegt;
c. die kopfformende Sonotrode (116, 116a, 116b) und der Stift (118, 118a, 118b) gleiten zurück weg von dem Tampon (162) mit geformtem Kopf.

9. Verfahren nach Anspruch 7, **dadurch gekennzeichnet, dass** das Formen des Einführendes (172) des Tampons (168), Schritt (c), ferner die Teilschritte umfasst:
a. der Stift (118, 118a, 118b) gleitet innerhalb des Trägerrohrs (106) nach vorn, stößt gegen den komprimierten Tampon (162) und drückt den Tampon (162) nach oben gegen die kopfformende Sonotrode (116, 116a, 116b), die sich in einer stationären Position befindet;
b. die kopfformende Sonotrode (116, 116a, 116b) überträgt Ultraschallschwingungen mit einer Frequenz zwischen 10 kHz und 70 kHz, vorzugsweise zwischen 20 kHz und 50 kHz, vorzugsweise von 35 kHz, während einer Zeitspanne, die zwischen 0,1 und 1 s, vorzugsweise zwischen 0,2 und 0,5 s, liegt, auf das Tampon-Einführende (172);
c. der Stift (118, 118a, 118b) gleitet zurück, und die kopfformende Sonotrode (116, 116a, 116b) gleitet nach vorn, so dass die kopfformende Sonotrode (116, 116a, 116b) den Tampon (162) zurück in seine Ausgangsposition im Trägerrohr (106) schiebt;
d. die kopfformende Sonotrode (116, 116a, 116b) gleitet nach hinten in ihre stationäre Position.

10. Verfahren nach Anspruch 8 oder 9, wobei:
a. das Einführende (172) des Tampons (162) durch die erste kopfformende Sonotrode (116a) unter Verwendung der in Anspruch 12 oder 13 beschriebenen Teilschritte geformt wird;
b. das Trägerrohr (106) und der Tampon (162) zur Position der zweiten kopfformenden Sonotrode (116b) befördert werden;
c. das Einführende (172) des Tampons (162) durch die zweite kopfformende Sonotrode (116b) unter Verwendung der in Anspruch 8 oder 9 beschriebenen Teilschritte geformt wird, wobei die für die erste und die zweite kopfformende Sonotrode (116a, 116b) verwendeten Teilschritte unterschiedlich sind.

## Revendications

1. Appareil (100) permettant de façonner l'extrémité d'introduction (172) d'un tampon (162), comprenant :
- un tube porteur (106) pour contenir le tampon (162) ;
- une roue de support (104) qui porte une pluralité de tubes porteurs (106), la roue de support (104) comprenant un moyen d'acheminement pour transporter chaque tube porteur (106) ; et
- une station de façonnage de tête (108) pour façonner l'extrémité d'introduction (172) du tampon (162) ;
la station de façonnage de tête (108) comprenant un dispositif à ultrasons (110), le dispositif à ultrasons (110) comprenant au moins une sonotrode de façonnage de tête (116) émettant des vibrations ultrasonores à une fréquence comprise entre 10 kHz et 70 kHz, de préférence entre 20 kHz et 50 kHz, de préférence de 35 kHz,
**caractérisé en ce que** le dispositif à ultrasons (110) comprend une première et une seconde sonotrode de façonnage de tête (116a, 116b) émettant des vibrations ultrasonores à une fréquence comprise entre 10 kHz et 70 kHz, de préférence entre 20 kHz et 50 kHz, de préférence de 35 kHz.

2. Appareil (100) selon la revendication 1, **caractérisé en ce que** les première et seconde sonotrodes de façonnage de tête (116a, 116b) sont alignées sur un axe arqué.

3. Appareil selon la revendication 1 ou 2, **caractérisé en ce que** la station de façonnage de tête (108) comprend un mécanisme d'actionnement (146) permettant le déplacement du dispositif à ultrasons (110) plus près ou plus loin de la roue de support (104) et du tampon (162).

4. Appareil selon la revendication 3 précédente, dans lequel l'appareil (100) comprend un mécanisme de poussée (112) comprenant une plaque (120) avec au moins une cheville saillante (118) pour appuyer sur l'extrémité arrière (168) du tampon (162) qui est opposée à l'extrémité d'introduction (172).

5. Appareil selon l'une quelconque des revendications précédentes, dans lequel les première et seconde sonotrodes de façonnage de tête (116a, 116b) ont des profils de section transversale différents.

6. Appareil selon l'une quelconque des revendications précédentes, dans lequel la sonotrode de façonnage de tête (116) a une cavité (136) en forme d'ogive.

7. Procédé de façonnage de l'extrémité d'introduction d'un tampon à l'aide de l'appareil (100) selon l'une quelconque des revendications précédentes, comprenant les étapes consistant à :
a. placer un tampon (162) à l'intérieur d'un tuyau porteur (106) ;
b. acheminer ledit tuyau porteur (106) et le tampon (162) vers une station de façonnage de tête (108) ;
c. façonner l'extrémité d'introduction (172) du tampon (162).
d. acheminer ledit tampon (162) loin de la station de façonnage de tête (108).
**caractérisé en ce que** le façonnage de l'extrémité d'introduction (172) du tampon (162) est effectué à l'aide d'un dispositif à ultrasons (110),
le dispositif à ultrasons (110) comprenant au moins une sonotrode de façonnage de tête (116) émettant des vibrations ultrasonores à une fréquence comprise entre 10 kHz et 70 kHz, de préférence entre 20 kHz et 50 kHz, de préférence de 35 kHz, pendant un laps de temps compris entre 0,1 et 1 s, de préférence entre 0,2 et 0,5 s,
**caractérisé en ce que** le dispositif à ultrasons (110) comprend une première et une seconde sonotrode de façonnage de tête (116a, 116b) émettant des vibrations ultrasonores à une fréquence comprise entre 10 kHz et 70 kHz, de préférence entre 20 kHz et 50 kHz, de préférence de 35 kHz, pendant un laps de temps compris entre 0,1 et 1 s, de préférence entre 0,2 et 0,5 s, le tampon (162) étant façonné par la première sonotrode de façonnage de tête (116a) puis ensuite par la seconde sonotrode de façonnage de tête (116b).

8. Procédé selon la revendication précédente, **caractérisé en ce que** le façonnage de l'extrémité d'introduction (172) du tampon (168), étape (c), comprend en outre les sous-étapes :
a. la sonotrode de façonnage de tête (116, 116a, 116b) et la cheville (118, 118a, 118b) glissent vers l'avant vers le tampon (162), la sonotrode de façonnage de tête (116, 116a, 116b) glisse pour venir en butée contre la tête du tampon (172) et la première cheville (118, 118a, 118b) glisse pour venir en butée contre l'extrémité arrière (168) du tampon ;
b. la sonotrode de façonnage de tête (116, 116a, 116b) émet des vibrations ultrasonores à une fréquence comprise entre 10 kHz et 70 kHz, de préférence entre 20 kHz et 50 kHz, de préférence de 35 kHz, pendant un laps de temps compris entre 0,1 et 1 s, de préférence entre 0,2 et 0,5 s.
c. la sonotrode de façonnage de tête (116, 116a, 116b) et la cheville (118, 118a, 118b) glissent vers l'arrière loin pour le tampon (162) en forme de tête.

9. Procédé selon la revendication 7, **caractérisé en ce que** le façonnage de l'extrémité d'introduction (172) du tampon (168), étape (c), comprend en outre les sous-étapes :
a. la cheville (118, 118a, 118b) glisse vers l'avant à l'intérieur du tube porteur (106) et vient buter contre le tampon (162) comprimé et pousse ledit tampon (162) contre la sonotrode de façonnage de tête (116, 116a, 116b) qui se trouve dans une position stationnaire ;
b. la sonotrode de façonnage de tête (116, 116a, 116b) applique des vibrations ultrasonores à l'extrémité d'introduction du tampon (172) à une fréquence comprise entre 10 kHz et 70 kHz, de préférence entre 20 kHz et 50 kHz, de préférence de 35 KHz, pendant un laps de temps compris entre 0,1 et 1 s, de préférence entre 0,2 et 0,5 s ;
c. la cheville (118, 118a, 118b) glisse vers l'arrière et la sonotrode de façonnage de tête (116, 116a, 116b) glisse vers l'avant de sorte que la sonotrode de façonnage de tête (116, 116a, 116b) repousse le tampon (162) dans sa position initiale dans le tube porteur (106) ;
d. la sonotrode de façonnage de tête (116, 116a, 116b) glisse vers l'arrière jusqu'à sa position stationnaire.

10. Procédé selon les revendications 8 ou 9, où :
a. l'extrémité d'introduction (172) du tampon (162) est façonnée par la première sonotrode de façonnage de tête (116a) à l'aide des sous-étapes selon la revendication 12 ou 13 ;
b. le tube porteur (106) et le tampon (162) sont acheminés vers la position de la seconde sonotrode de façonnage de tête (116b) ;
c. l'extrémité d'introduction (172) du tampon (162) est façonnée par la seconde sonotrode de façonnage de tête (116b) à l'aide des sous-étapes selon la revendication 8 ou 9, les sous-étapes utilisées pour les première et seconde sonotrodes de façonnage de tête (116a, 116b) étant différentes.
